(19) Europäisches Patentamt
European Patent Office
Office européen des brevets

(11) EP 4 328 293 B1

(12) **EUROPEAN PATENT SPECIFICATION**

(45) Date of publication and mention of the grant of the patent:
**14.01.2026 Bulletin 2026/03**

(21) Application number: **22876676.2**

(22) Date of filing: **24.08.2022**

(51) International Patent Classification (IPC):
***C12M 1/12*** *(2006.01)* ***C12N 11/06*** *(2006.01)*
***C12N 11/08*** *(2020.01)* ***A61K 8/98*** *(2006.01)*
***A61K 9/16*** *(2006.01)* ***A61K 35/12*** *(2015.01)*
***A61K 8/73*** *(2006.01)* ***A61K 8/02*** *(2006.01)*
***A61K 8/65*** *(2006.01)* ***A61K 47/69*** *(2017.01)*

(52) Cooperative Patent Classification (CPC):
**A61Q 19/00; A61K 8/64; A61K 8/65; A61K 8/735; A61K 9/0019; A61K 9/0024; A61K 35/12; A61K 47/36; A61K 47/42; A61L 27/38; C12N 11/06; C12N 11/08;** A61K 2800/412; A61L 2300/622; A61L 2300/64

(86) International application number:
**PCT/KR2022/012635**

(87) International publication number:
**WO 2023/054901 (06.04.2023 Gazette 2023/14)**

(54) **MICRO CARRIER, CELL COMPOSITE, AND MEDICAL COMPOSITION; COSMETIC COMPOSITION; MEDICAL ARTICLES AND COSMETIC ARTICLES USING THE SAME**

MIKROTRÄGER, ZELLVERBUNDSTOFF UND MEDIZINISCHE ZUSAMMENSETZUNG, KOSMETISCHE ZUSAMMENSETZUNG, MEDIZINISCHE ARTIKEL UND KOSMETISCHE ARTIKEL, DIE DIESE VERWENDEN

MICROSUPPORT, COMPOSITE CELLULAIRE, COMPOSITION MÉDICALE, COMPOSITION COSMÉTIQUE, ARTICLES MÉDICAUX ET ARTICLES COSMÉTIQUES LES UTILISANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.09.2021 KR 20210129845**

(43) Date of publication of application:
**28.02.2024 Bulletin 2024/09**

(73) Proprietor: **LG Chem, Ltd.**
**Seoul 07336 (KR)**

(72) Inventors:
- **KIM, Yunseop**
  **Daejeon 34122 (KR)**
- **KIM, Yeji**
  **Daejeon 34122 (KR)**
- **KIM, Jee Seon**
  **Daejeon 34122 (KR)**

(74) Representative: **Plasseraud IP**
**104 Rue de Richelieu**
**CS92104**
**75080 Paris Cedex 02 (FR)**

(56) References cited:
**KR-A- 20030 036 081**
**KR-A- 20210 011 340**
**KR-A- 20210 036 463**
**KR-A- 20210 037 578**
**KR-A- 20210 121 576**
**US-A- 5 856 464**
**US-A1- 2003 185 798**
**US-A1- 2012 183 616**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 328 293 B1

## Description

## [TECHNICAL FIELD]

CROSS-REFERENCE TO RELATED APPLICATION(S)

This application claims the benefit of Korean Patent Application No. 10-2021-0129845 filed on September 30, 2021 with the Korean Intellectual Property Office.

The present invention relates to a micro carrier that not only can achieve stability together with high crosslinking efficiency and production yield, but also can confirm drug release properties without chemical modification of protein or peptide pharmaceutical effective materials, can be injected into the body immediately after 3D culture without a cell detachment process and can provide a stable environment for adherent cells, thereby increasing cell viability and contributing to a high implantation rate in the body, and a cell composite, medical composition, cosmetic composition, medical articles and cosmetic articles using the same.

## [BACKGROUND ART]

Drug delivery system refers to a series of techniques that enable the delivery and release of a drug to a target site while maintaining the activity of the drug. As the population is aging, the needs for health care and drug treatment are increasing, and drug delivery techniques are actively developed to meet the needs.

The drug delivery system can be classified into oral dosage forms, transdermal dosage forms, injection forms, pulmonary inhalation dosage forms, and mucosal dosage forms according to the delivery routes. The oral dosage form is the most common drug delivery method administered orally and occupies the largest share in the market. The transdermal dosage form is a method of delivering drugs through the skin, which can be administered continuously for extended period of time and facilitates control of blood levels. The injection form are divided into intravenous, intramuscular, and subcutaneous injections, and the pulmonary inhalation and mucosal administration have the advantage of being absorbed quickly and avoiding pain. The carrier used in the field of such drug delivery system must ensure biocompatibility, and therefore, attempts are continuously made to utilize natural polymers. Further, the drugs used have been expanded from low-molecular-weight synthetic drugs to high-molecular-weight protein or peptide drugs. The drug release model of the drug delivery system is a model for observing drug release phenomena over time based on the system parameters. A mathematical model of drug release can be a tool for determining and predicting how each parameter in a drug delivery system affects the release behavior, and is therefore of great importance because it enables the design of novel drug delivery systems. In this process, it is necessary to accurately measure and analyze the degree of drug release over time in each model.

Hyaluronic acid and gelatin, which are biocompatible polymers, are used as polymeric materials for drug delivery system carriers. Hyaluronic acid, which is mainly used as a biocompatible material, is composed of N-acetyl-D-glucosamine and D-glucuronic acid, and is a biopolymer material in which the repeating units are linearly connected. This is abundantly present in the vitreous humor of the eye, synovial fluid of joints, cockscomb, and the like. Hyaluronic acid is often used as a bioinjectable material due to its excellent biocompatibility and viscoelasticity, but hyaluronic acid itself is easily decomposed in vivo or under conditions such as acids and alkalis, which limits its use. Meanwhile, gelatin is a polymer obtained by hydrolyzing collagen, which is a biological connective tissue, and is often used as a scaffold for cell culture. Cells can be collected or cultured using the cell adhesion performance of gelatin, but it is weak in strength and has phase transition characteristics depending on temperature, and thus, studies have been made to improve the physical properties by introducing functional groups by chemical methods.

Numerous publications concerning the drug delivery systems using hyaluronic acid and gelatin have proposed chemical crosslinking of hyaluronic acid/gelatin in order to improve the physical/chemical stability of the material against the body environment, and chemical crosslinking mainly proceeds through crosslinking between hydroxyl functional groups or carboxylates of hyaluronic acid and amines or carboxylates, which are residues of amino acids in gelatin. However, in the case of deliverable protein or peptide active materials, most of them possess functional groups such as hydroxyl and amine, similar to the constituents of polymer particles, and chemically bond to the carrier during the crosslinking process, which makes it difficult to confirm the actual drug release behavior. Document KR 2021-0037578 discloses polymer microparticles comprising a polymer matrix formed by crosslinking a biocompatible polymer (gelatin and hyaluronic acid) via crosslinking agents (2 crosslinking steps), wherein the polymer microparticles are usable as a microcarrier or a drug carrier for effective drug delivery of drugs applied to the human body.

Therefore, there is a need to develop micro carriers or polymer micro particles capable of confirming drug release properties without causing structural changes in the protein and peptide pharmaceutical active materials to be delivered.

Further, in the process of large-scale expansion of adherent cells using micro carriers, a spherical micro carrier that can increase surface efficiency has been developed to overcome the limitations of existing two-dimensional culture, and utilized for three-dimensional expansion culture. In the prior art, in which cells are recovered through a cell detachment process after the completion of culture, there are problems of an increase in manufacturing cost and cell damage due to the addition of a detachment process.

Further, in the case of injectable cell therapeutic agent, the cells are mainly injected into the affected area in a state of floating on the liquid phase, whereby in the case of adherent cells, there was a limitation in that the transplantation rate and cell viability are lowered due to an unstable environment for survival and an immune response in the body.

Therefore, there is a need to develop a micro carrier that can be injected into the body immediately after 3D culture without a cell detachment process and can provide a stable environment for adherent cells, thereby increasing cell viability and contributing to a high implantation rate in the body.

## [DETAILED DESCRIPTION OF THE INVENTION]

### [Technical Problem]

It is an object of the present invention to provide a micro carrier that not only can achieve stability together with high crosslinking efficiency and production yield, but also can confirm drug release properties without chemical modification of protein or peptide pharmaceutical effective materials, can be injected into the body immediately after 3D culture without a cell detachment process and can provide a stable environment for adherent cells, thereby increasing cell viability and contributing to a high implantation rate in the body.

It is another object of the present invention to provide a cell composite comprising the micro carrier.

It is another object of the present invention to provide a medical composition comprising the micro carrier or the cell composite.

It is a further object of the present invention to provide a cosmetic composition comprising the micro carrier or the cell composite.

It is still another object of the present invention to provide medical articles comprising the medical composition.

It is still further another object of the present invention to provide cosmetic articles comprising the cosmetic composition.

### [Technical Solution]

There is provided a micro carrier comprising polymer micro particles which comprises: a polymer matrix containing a biocompatible polymer; and a polypeptide dispersed in the polymer matrix and having a reactive functional group content of 1 mol/$10^3$ g or less, wherein the biocompatible polymer comprises hyaluronic acid and gelatin, wherein the polypeptide having the reactive functional group content of 1 mol/$10^3$ g or less comprises a polypeptide in which 90 mol% or more of reactive functional groups on the surface are substituted with a blocking compound, wherein the reactive functional group is an amine group, wherein the reactive functional group content is calculated according to the following Equation, [Equation] Reactive amine functional group content (mol/$10^3$ g) = Amount of hydrochloric acid added (L) x Molarity of aqueous hydrochloric acid solution (mol/ L) / Amount of polypeptide dissolved in solvent ($10^3$ g), as defined in the claims.

In another aspect, there is provided a cell composite as defined in claim 9.

In another aspect, there is provided a medical as defined in claim 10.

In a further aspect, there is provided a cosmetic composition as defined in claim 11.

In still another aspect, there is provided a medical article according to claim 12.

In still further another aspect, there is provided a cosmetic article according to claim 12.

A micro carrier, a cell composite, medical composition, cosmetic composition, medical articles and cosmetic articles using the same according to specific embodiments of the present invention will be described in more detail below.

The singular forms "a," "an" and "the" used herein are intended to include plural forms, unless the context clearly indicates otherwise.

It should be understood that the terms "comprise," "include", "have", etc. are used herein to specify the presence of stated feature, region, integer, step, action, element and/or component, but do not preclude the presence or addition of one or more other feature, region, integer, step, action, element, component and/or group.

Further, the terms including ordinal numbers such as "a first", "a second", etc. are used only for the purpose of distinguishing one component from another component, and are not limited by the ordinal numbers. For instance, a first component may be referred to as a second component, or similarly, the second component may be referred to as the first component, without departing from the scope of the present invention.

As used herein, the term "substituted or unsubstituted" means being unsubstituted or substituted with one or more substituents selected from the group consisting of deuterium; a halogen group; a cyano group; a nitro group; a hydroxy group; a carbonyl group; an ester group; an imide group; an amide group; a primary amino group; a carboxyl group; a sulfonic acid group; a sulfonamide group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkoxysilylalkyl group; an aryl phosphine group; and a heterocyclic group containing at least one of N, O and S atoms, or being unsubstituted or substituted with a substituent to which two or more substituents of the above-exemplified substituents are connected. For example, "a substituent in which two or more substituents are connected" may be a biphenyl group. Namely, a biphenyl group may be an aryl group, or it may also be interpreted as a substituent in which two phenyl groups are connected.

As used herein, the (co)polymer includes both a polymer or a copolymer, the polymer means a homopolymer consisting of a single repeating unit, and the copolymer means a composite polymer containing two or more repeating units.

The molecular weight of the polymer used herein is in accordance with the manufacturer's measurement method, and the method does not deviate from the conventional GPC measurement method. For example, in the present specification, the weight average molecular weight may mean a molecular weight measured by a light scattering method or a viscosity method.

While the present invention can be modified in various ways and take on various alternative forms, specific embodiments thereof are illustrated and described in detail below. However, it should be understood that there is no intent to limit the present invention to the particular forms disclosed, but on the contrary, the intention is to cover all modifications, equivalents, and alternatives falling within the spirit and scope of the invention as described.

As used herein, the term "micro particles" means that the cross section of the particles is circular or elliptical, and the minor axis/major axis ratio (spheroidization ratio) of the particles is in the range of 0.7 to 1.0. The length of the minor axis and the major axis of the particles can be derived by taking an optical photograph of the particles and calculating the average value of 30 to 100 arbitrary particles in the optical photograph.

As used herein, an emulsion means a mixed phase in which one or more of immiscible liquids of an oil phase or an aqueous phase is dispersed in a fine particle state (dispersoid) in another liquid (dispersion medium). Emulsions can be generally divided into macro emulsions, micro emulsions, and nano emulsions depending on the particle size of the dispersed phase.

Now, the present invention will be described in more detail.

## 1. Micro carrier

According to one embodiment of the invention, there can be provided a micro carrier comprising polymer micro particles which comprises: a polymer matrix containing a biocompatible polymer; and a polypeptide dispersed in the polymer matrix and having a reactive functional group content of 1 mol/$10^3$ g or less, wherein the biocompatible polymer comprises hyaluronic acid and gelatin, wherein the polypeptide having the reactive functional group content of 1 mol/$10^3$ g or less comprises a polypeptide in which 90 mol% or more of reactive functional groups on the surface are substituted with a blocking compound, wherein the reactive functional group is an amine group, wherein the reactive functional group content is calculated according to the following Equation, [Equation] Reactive amine functional group content (mol/103 g) = Amount of hydrochloric acid added (L) x Molarity of aqueous hydrochloric acid solution (mol/L) / Amount of polypeptide dissolved in solvent ($10^3$ g)

In conventional polymer micro particles, the crosslinking reaction proceeds by adding the particles directly onto an organic solvent, the morphology of the particles is greatly influenced by the concentration of the polymer and the content of the crosslinking agent, especially when the concentration of the polymer is low or the content of the crosslinking agent is small, there is a problem that the morphology of the particles cannot be maintained, which makes it difficult to form micro particles.

Thus, the present inventors have maximized the crosslink density with the same amount of crosslinking agent as the crosslinking reaction proceeds two times as in the production method of polymer micro particles described later, and at the same time, confirmed the release behavior of the pharmaceutically active material within the micro particles, thereby completing the present invention.

In particular, the inventors also confirmed through experiments that as the secondary crosslinking reaction proceeds in a solid phase during the secondary crosslinking reaction, spherical particles can be realized even with a small amount of crosslinking agent, and the production yield is improved.

The inventors further confirmed through experiments that as the micro carrier of one embodiment further comprises a cell adhesion inducing layer formed on the surface of the polymer micro particles, it can be injected into the body immediately after 3D culture without cell detachment process, and adherent cells form a composite in a state of being stably attached to the micro carrier, which thus increases the cell viability and realizes a high implantation rate in the body, thereby completing the invention.

The biocompatible polymer comprises a mixture of hyaluronic acid and gelatin.

As used herein, the hyaluronic acid may include both hyaluronic acid itself and hyaluronate. Thereby, the aqueous hyaluronic acid solution may be a concept that includes all of an aqueous solution of hyaluronic acid, an aqueous solution of hyaluronate, and a mixed aqueous solution of hyaluronic acid and hyaluronate. The hyaluronate may be inorganic salts such as sodium hyaluronate, potassium hyaluronate, calcium hyaluronate, magnesium hyaluronate, zinc hyaluronate, and cobalt hyaluronate, organic salts such as hyaluronic acid tetrabutylammonium, and mixtures thereof.

In one embodiment of the invention, the molecular weight of hyaluronic acid is not particularly limited, but is preferably 500 g/mol or more and 5,000,000 g/mol or less in order to realize various physical properties and biocompatibility.

As used herein, the gelatin may mean a protein obtained by treating collagen derived from animals with acid or alkali. followed by extraction.

In one embodiment of the invention, the molecular weight of gelatin is not particularly limited, but is preferably 100,000 g/mol or more and 5,000,000 g/mol or less in order to realize various physical properties and biocompatibility.

The present inventors have conducted intensive research on polymer micro particles, confirmed that as will be described later, the secondary crosslinking reaction proceeds in the dehydrated and aggregated solid phase after the primary crosslinking reaction in the swollen emulsion, thereby maximizing the crosslinking density with the same amount of crosslinking agent, and completed the present invention.

In particular, the inventors have confirmed through experiments that as the secondary crosslinking reaction among the two crosslinking reactions proceeds in a more aggregated solid phase, spherical particles can be formed even with a small amount of crosslinking agent, and the production yield is improved, and completed the present invention.

In addition, the inventors have confirmed through experiments that in the case of conventionally used polymeric micro particles, a pharmaceutically active material participates in crosslinking during production, which causes technical problems such as changes in structure and loss or deterioration of the actual release characteristics, but in the case of the polymer micro particles of the present invention, excellent properties as a protein drug release model can be realized by using a polypeptide dispersed in the polymer matrix and having a reactive functional group content of 1 mol/$10^3$g or less, and completed the present invention.

Specifically, the polymer micro particles contained in the micro carrier of the one embodiment includes a polypeptide having a reactive functional group content of 1 mol/$10^3$ g or less, or 0.001 mol/$10^3$g or more and 1 mol/$10^3$g or less, or 0.001 mol/$10^3$g or more and 0.5 mol/$10^3$g or less, or 0.001 mol/$10^3$g or more and 0.1 mol/$10^3$g or less.

That is, in the one embodiment, the reactive functional group content of the polypeptide being 1 mol/$10^3$ g or less may mean that the reactive functional group is contained in an amount of 1 mol or less with respect to $10^3$ g of the polypeptide contained in the polymer micro particles contained in the micro carrier of the one embodiment.

The method for measuring the reactive functional group content is not particularly limited, but for example, the content can be measured by dissolving $10^3$ g of polypeptide in an organic solvent, adding an indicator such as methyl red or phenolphthalein, and then performing neutralization titration with 0.1 M hydrochloric acid (amine functional group) or sodium hydroxide (carboxyl functional group), or can be calculated according to the following Equation.

Reactive amine functional group content (mol/$10^3$ g) = Amount of hydrochloric acid added (L) x Molarity of aqueous hydrochloric acid solution (mol/L) / Amount of polypeptide dissolved in solvent ($10^3$ g) [Equation]

As the polymer micro particles contained in the micro carrier of one embodiment includes a polypeptide having a reactive functional group content of 1 mol/$10^3$g or less, the reactive functional group is absent or contained in trace amounts in the polypeptide, so that reactions between the crosslinking agent and the reactive functional group within the polypeptide can be minimized during the process of chemical crosslinking to improve the physical and chemical stability of polymer micro particles. Thereby, the problem of loss of function of the protein drug due to crosslinking does not occur, and excellent protein drug release properties can be realized.

In one embodiment, the hydrodynamic radius of the polypeptide may be 1 nm to 30 nm, or 1 nm to 20 nm, or 1 nm to 15 nm.

As the hydrodynamic radius of the polypeptide is 1 nm to 30 nm, technical effects can be realized that can adjust the release rate of a polypeptide from the same polymer microparticle.

Specifically, the hydrodynamic radius of the polypeptide can be measured by placing 3 mL of a polypeptide aqueous solution with a concentration of 1 mg/mL in a plastic square cuvette and using a NanoBrook 173 plus instrument (Brookhaven Instruments Corporation) at 37°C.

The polypeptide having a reactive functional group content of 1 mol/$10^3$ g or less in the one embodiment comprises a polypeptide in which 90 mol% or more, 90 mol% or more and 100 mol% or less, or 95 mol% or more and 100 mol% or less, or 99 mol% or more and 100 mol% or less of the reactive functional groups on the surface are substituted with a blocking compound.

Examples of the blocking compound are not particularly limited, and any compound having a functional group capable of reacting with a reactive functional group contained in the polypeptide to form a chemical bond and a blocking group having weak reactivity can be used without limitation.

An example of a functional group capable of reacting with a reactive functional group contained in the polypeptide to form a chemical bond may include an isocyanate group, a carboxy group, a halogen group, and the like. In addition, an example of the blocking group having weak reactivity include an alkyl group having 1 to 50 carbon atoms, a cycloalkyl group having 2 to 50 carbon atoms, an aryl group having 6 to 50 carbon atoms, a heteroaryl group having 3 to 50 carbon atoms, and the like.

As used herein, the alkyl group is a monovalent functional group derived from an alkane, and may be a straight-chain or a branched-chain, and the number of carbon atoms in the straight-chain alkyl group is not particularly limited, but is preferably 1 to 20. In addition, the number of carbon atoms of the branched chain alkyl group is 3 to 20. Specific examples of the alkyl group include methyl, ethyl, propyl, n-propyl, isopropyl, butyl, n-butyl, isobutyl, tert-butyl, sec-butyl, 1-methyl-butyl, 1-ethyl-butyl, pentyl, n -pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, octyl, n-octyl, tert-octyl, 1-methylheptyl, 2-ethylhexyl, 2-propylpentyl, n-nonyl, 2,2-dimethylheptyl, 1-ethyl- propyl, 1,1-dimethyl-propyl, isohexyl, 4-methylhexyl, 5-methylhexyl, 2,6-dimethylheptan-4-yl, and the like. The alkyl group may be substituted or unsubstituted, and when substituted, examples of the substituent are as described above.

As used herein, a cycloalkyl group is a monovalent functional group derived from a cycloalkane, and may be a monocyclic group or a polycyclic group, but is not particularly limited and the number of carbon atoms may be 3 to 20. According to one embodiment, the number of carbon atoms of the cycloalkyl group is 3 to 10. Specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, biscyclo[2,2,1]heptyl, and the like, but are not limited thereto. The cycloalkyl group may be substituted or unsubstituted, and when substituted, examples of the substituent are as described above.

As used herein, the aryl group is a monovalent functional group derived from an arene, and is not particularly limited, but preferably has 6 to 20 carbon atoms, and may be a monocyclic group or a polycyclic group. The monocyclic aryl group may include, but not limited to, a phenyl group, a biphenyl group, a terphenyl group, or the like. The polycyclic aryl group may include, but not limited to, a naphthyl group, an anthracenyl group, a phenanthryl group, a pyrenyl group, a perylenyl group, a chrycenyl group, a fluorenyl group or the like. The aryl group may be substituted or unsubstituted, and when substituted, examples of the substituent are as described above.

As used herein, a heteroaryl group includes one or more atoms other than carbon, that is, one or more heteroatoms, and specifically, the heteroatom may include one or more atoms selected from the group consisting of O, N, Se, and S, and the like. The number of carbon atoms thereof is not particularly limited, but is preferably 4 to 20, and the heteroaryl group may be monocyclic or polycyclic. Examples of a heterocyclic group include a thiophene group, a furanyl group, a pyrrole group, an imidazolyl group, a thiazolyl group, an oxazolyl group, an oxadiazolyl group, a pyridyl group, a bipyridyl group, a pyrimidyl group, a triazinyl group, a triazolyl group, an acridyl group, a pyridazinyl group, a pyrazinyl group, a qinolinyl group, a quinazolinyl group, a quinoxalinyl group, a phthalazinyl group, a pyridopyrimidyl group, a pyridopyrazinyl group, a pyrazinopyrazinyl group, an isoquinolinyl group, an indolyl group, a carbazolyl group, a benzoxazolyl group, a benzimidazolyl group, a benzothiazolyl group, a benzocarbazolyl group, a benzothiophene group, a dibenzothiophene group, a benzofuranyl group, a phenanthrolinyl group (phenanthroline), a thiazolyl group, an isoxazolyl group, an oxadiazolyl group, a thiadiazolyl group, a benzothiazolyl group, a phenothiazinyl group, an aziridyl group, an azaindolyl group, an isoindolyl group, an indazolyl group, a purine group (purine), a pteridyl group (pteridine), a beta-carboline group, a naphthyridyl group (naphthyridine), a terpyridyl group, a phenazinyl group, an imidazopyridyl group, a pyropyridyl group, an azepine group, a pyrazolyl group, a dibenzofuranyl group, and the like, but are not limited thereto. The heteroaryl group may be substituted or unsubstituted, and when substituted, examples of the substituent are as described above.

Examples of halogen groups are fluorine, chlorine, bromine or iodine.

More specifically, examples of the blocking compound is a blocking compound having a functional group containing electrophilic carbon, and may include one or more compounds selected from the group consisting of Fluorescein isothiocyanate (FITC), Rhodamine B isothiocyanate (RBITC), Tetramethylrhodamine B isothiocyanate, N-hydroxy-succinimidyl-ester linked fluorescein (NHS-fluorescein), 6-[Fluorescein-5(6)-carboxamido]hexanoic acid N-hydroxysuccinimide ester, Fluorescein-5-EX N-hydroxysuccinimide ester, Alexa Fluor™ 350 NHS Ester, Alexa Fluor™ 430 NHS Ester, Alexa Fluor™ 488 NHS Ester, Rhodamine Green™ Carboxylic Acid, Succinimidyl Ester, Hydrochloride (5(6)-CR 110, SE), Rhodamine Red™-X, 5-isomer, and Alexa Fluor™ 647 NHS Ester.

As the reactive functional group of the polypeptide reacts with the blocking compound and is substituted by 90 mol% or more, the content of the reactive functional group of the polypeptide contained in the polymer micro particles contained in the micro carrier of the one embodiment may be 1 mol/$10^3$g or less as described above.

That is, as the reactive functional group of the polypeptide reacts with the blocking compound and is substituted by 90 mol% or more, the reactive functional group is absent or contained in trace amounts in the polypeptide, so that reactions between the crosslinking agent and the reactive functional group can be minimized during the chemical crosslinking process to improve the physical and chemical stability of the polymer micro particles. Thereby, the problem of loss of function of the protein drug due to crosslinking does not occur, and excellent protein drug release properties can be realized.

The reactive functional group is an amine group.

The reactive functional group may be a reactive functional group contained in the polypeptide contained in the polymer microparticle.

As the reactive functional group reacts with the crosslinking agent during the chemical crosslinking process to improve physical and chemical stability, there may be a problem that the protein drug function is lost due to crosslinking, and the protein drug release characteristic is not realized.

In one embodiment, the polypeptide may include a polypeptide having a functional group that is a combination of a blocking compound and a reactive functional group. That is, the polypeptide may have a functional group that is a combination of a blocking compound and a reactive functional group, and a reactive functional group that does not react with the blocking compound. In addition, the content of the reactive functional group that did not react with the blocking compound in the polypeptide may satisfy 1 mol/$10^3$ g or less as described above.

Examples of the functional group that is a combination of the blocking compound and the reactive functional group are not particularly limited, and any functional group contained in the reaction product obtained by the reaction of the above-mentioned various blocking compounds with the reactive functional group may be applied without limitation. An example of a functional group that is a combination of the blocking compound and a reactive functional group may include urethane, thiourethane, urea, thiourea, amide, ether, thioether, amine, and the like.

In a more specific example, the polypeptide may include a polypeptide having a thiourea or thiourethane-based functional group, which is a combination of a blocking compound containing an isothiocyanate group and a reactive functional group.

Specifically, the polymer micro particles may include a reaction product of a blocking compound containing isothiocyanate group and a polypeptide containing a reactive functional group.

That is, the polymer may include a polypeptide having a thiourea-based functional group in which an isothiocyanate group (-N=C=S) contained in the blocking compound containing isothiocyanate group, and a reactive functional group contained in a polypeptide containing a reactive functional group react to form a thiourea-based functional group.

Further, examples of the polypeptide containing the reactive functional group are not particularly limited, but depending on the application use of the polymer micro particles of the one embodiment, any polypeptide suitable for the application can be applied without limitation. That is, specific examples of the polypeptide are not limited, and may include a primary amine at the N-terminus. For example, the polypeptide may be polyglycine, polyalanine, polyvaline, polyleucine, polyisoleucine, polyphenylalanine, polytryptophan, polyglutamic acid, polyproline, or polyaspartic acid.

When the polypeptide contains 1 mol/$10^3$ g or more of a nucleophilic functional group other than a primary amine, not all reactive functional groups are protected even when reacting with a blocking compound, so that the drug release properties of the polymer micro particles may be deteriorated due to the addition reaction of the chemical crosslinking agent and the unprotected reactive functional group.

The addition amount of the polypeptide is also not particularly limited, and the content can be used without limitation depending on the application purpose and object. For example, the polypeptide may be contained in an amount of 0.0001 parts by weight or more and 1000 parts by weight or less based on 100 parts by weight of the polymer micro particles, which can be contained without limitation in a small amount or an excess amount relative to the polymer micro particles.

Meanwhile, the polymer matrix may include a first crosslinking region in which the biocompatible polymer is crosslinked via a first crosslinking agent; and a second crosslinking region in which the biocompatible polymer is crosslinked via a second crosslinking agent.

The first crosslinking region means a crosslinking region formed through a first crosslinking reaction between the emulsion containing the biocompatible polymer and the first crosslinking agent, and the second crosslinking region may mean a crosslinking region formed by the progress of a secondary crosslinking reaction with the second crosslinking agent, without proceeding a first crosslinking reaction with a first crosslinking agent in the emulsion containing the biocompatible polymer, and a crosslinking region formed through an additional secondary crosslinking reaction with the first crosslinking region and the second crosslinking agent.

In one embodiment of the present invention, examples of the first crosslinking agent are not particularly limited. Specifically, the first crosslinking agent may include one of iron (III) chloride and aluminum chloride.

In one embodiment of the present invention, examples of the second crosslinking agent are not particularly limited. Specifically, the second crosslinking agent may include at least one selected from the group consisting of 1,4-butandiol diglycidyl ether (BDDE), ethylene glycol diglycidyl ether (EGDGE), 1,6-hexanediol diglycidyl ether, propylene glycol diglycidyl ether, polypropylene glycol diglycidyl ether, polytetramethylene glycol diglycidyl ether, neopentyl glycol diglycidyl ether, polyglycerol polyglycidyl ether, diglycerol polyglycidyl ether, glycerol polyglycidyl ether, tri-methylpropane polyglycidyl ether, 1,2-(bis(2,3-epoxypropoxy)ethylene, pentaerythritol polyglycidyl ether, sorbitol polyglycidyl ether, divinylsulfone and glutaraldehyde. More specifically, the second crosslinking agent may be 1,4-butandiol diglycidyl ether (BDDE).

Meanwhile, the polymer micro particles contained in the micro carrier may have an average diameter of 1 $\mu$m or more and 1000 $\mu$m or less, 100 $\mu$m or more and 1000 $\mu$m or less, 100 $\mu$m or more and 800 $\mu$m or less, 100 $\mu$m or more and 750 $\mu$m or less, 100 $\mu$m or more and 600 $\mu$m or less, 150 $\mu$m or more and 600 $\mu$m or less in distilled water. When the average diameter of the polymer micro particles satisfies the above-mentioned range, cell adhesion and culture performance are excellent.

Further, the polymer micro particles have a swelling degree of 10 or less, 0.1 or more and 10 or less, 0.1 or more and 5 or less, 0.5 or more and 5 or less, 0.9 or more and 4.5 or less according to the following Equation 1:

Swelling degree = {(Average diameter in distilled water)$^3$ - (Average diameter of dried particles)$^3$} / (Average diameter of dried particles)$^3$. [Equation 1]

When the swelling degree of the polymer micro particles exceeds 10, there may be a technical problem that the crosslinking density is low, the mechanical strength is inferior, and the stability decreases.

Further, the polymer micro particles contained in the micro carrier of the one embodiment may have a release amount of the pharmaceutically active material under release conditions of pH 5.0 or more and 30°C or more and 40°C or less after culturing for more than 150 hours or more, of 20% or more and 100% or less, 50% or more and 100% or less, 75% or more and 100% or less, 90% or more and 100%.

The release conditions may be specifically pH 5.0 or more, pH 5.0 or more and pH 14.0 or less, pH 5.0 or more and pH 11.0 or less, and pH 5.6 or more and pH 11.0 or less.

Further, the release conditions may be specifically 30°C or more and 40°C or less, 35°C or more and 40°C or less, and 36°C or more and 38°C or less.

As the release amount of the polymer micro particles contained in the micro carrier of one embodiment is 20% or more and 100% or less, a crosslinking agent can be used to realize polymer micro particles having excellent drug active material release properties.

Examples of the method for producing the polymer micro particles contained in the micro carrier of the one embodiment is not particularly limited, but for example, a method for producing polymer micro particles compirsing the step of: subjecting a mixture containing a combination of a polypeptide and a blocking compound, a biocompatible polymer, and a first crosslinking agent to a primary crosslinking reaction to form crosslinked particles; and extracting the crosslinked particles and performing a secondary crosslinking reaction in an organic solvent containing a second crosslinking agent can be used.

In conventional polymer micro particles, the crosslinking reaction proceeds by adding the particles directly onto an organic solvent, the morphology of the particles is greatly influenced by the concentration of the polymer and the content of the crosslinking agent, especially when the concentration of the polymer is low or the content of the crosslinking agent is small, there is a problem that the morphology of the particles cannot be maintained, which makes it difficult to form micro particles.

Therefore, the present inventors have maximized the crosslink density with the same amount of crosslinker as the crosslinking reaction proceeds two times similarly to the production method of polymer micro particles described later, and at the same time, confirmed the release behavior of the pharmaceutically active material.

In particular, the inventors also confirmed through experiments that as the secondary crosslinking reaction proceeds in the solid phase during the secondary crosslinking reaction, spherical particles can be realized even with a small amount of crosslinking agent, and the production yield is improved.

The biocompatible polymer comprises a mixture of hyaluronic acid and gelatin.

As used herein, the hyaluronic acid may include both hyaluronic acid itself and hyaluronate. Therefore, the aqueous hyaluronic acid solution may be a concept that includes all of an aqueous solution of hyaluronic acid, an aqueous solution of hyaluronate, and a mixed aqueous solution of hyaluronic acid and hyaluronate. The hyaluronate may be inorganic salts such as sodium hyaluronate, potassium hyaluronate, calcium hyaluronate, magnesium hyaluronate, zinc hyaluronate, and cobalt hyaluronate, organic salts such as hyaluronic acid tetrabutylammonium, and mixtures thereof.

In one embodiment of the invention, the molecular weight of hyaluronic acid is not particularly limited, but is preferably 500 g/mol or more and 5,000,000 g/mol or less in order to implement various physical properties and biocompatibility.

As used herein, the gelatin may mean a protein obtained by treating collagen derived from animals with acid or alkali, followed by extraction.

In one embodiment of the invention, the molecular weight of gelatin is not particularly limited, but is preferably 100,000 g/mol or more and 5,000,000 g/mol or less in order to realize various physical properties and biocompatibility.

In the combination of the polypeptide and the blocking compound, examples of the blocking compound are not particularly limited, and any compound having a functional group capable of reacting with a reactive functional group contained in the polypeptide to form a chemical bond and a blocking group having weak reactivity can be used without limitation.

An example of a functional group capable of reacting with a reactive functional group contained in the polypeptide to form a chemical bond may include an isocyanate group, a carboxy group, a halogen group, and the like. In addition, an example of the blocking group having weak reactivity include an alkyl group having 1 to 50 carbon atoms, a cycloalkyl group having 2 to 50 carbon atoms, an aryl group having 6 to 50 carbon atoms, a heteroaryl group having 3 to 50 carbon atoms, and the like.

More specifically, examples of the blocking compound is a blocking compound having a functional group containing electrophilic carbon, and may include one or more compounds selected from the group consisting of Fluorescein isothiocyanate (FITC), Rhodamine B isothiocyanate (RBITC), Tetramethylrhodamine B isothiocyanate, N-hydroxy-succinimidyl-ester linked fluorescein (NHS-fluorescein), 6-[Fluorescein-5(6)-carboxamido]hexanoic acid N-hydroxysuccinimide ester, Fluorescein-5-EX N-hydroxysuccinimide ester, Alexa Fluor™ 350 NHS Ester, Alexa Fluor™ 430 NHS Ester, Alexa Fluor™ 488 NHS Ester, Rhodamine Green™ Carboxylic Acid, Succinimidyl Ester, Hydrochloride (5(6)-CR 110, SE), Rhodamine Red™-X, 5-isomer, and Alexa Fluor™ 647 NHS Ester.

In addition, examples of the polypeptide are not particularly limited, but depending on the application use of the polymer micro particles of the one embodiment, any polypeptide suitable for the use can be applied without limitation. That is, specific examples of the polypeptide are not limited, and may include one or less primary amines. For example, the polypeptide may be polyglycine, polyalanine, polyvaline, polyleucine, polyisoleucine, polyphenylalanine, polytryptophan, polyglutamic acid, polyproline, or polyaspartic acid.

When the polypeptide contains two or more primary amines, not all reactive functional groups are protected with thiourea-based functional groups even when reacting with a blocking compound containing isothiocyanate group, and thus it contains a reactive functional group that is not protected with a thiourea-based functional group, so that the drug release properties of the polymer micro particles can be deteriorated due to an addition reaction of the unprotected reactive functional group.

In a more specific example, the combination of the polypeptide and the blocking compound may include a combination of a polypeptide having a thiourea or thiourethane-based functional group, which is a combination of a blocking compound containing an isothiocyanate group and a reactive functional group.

Specifically, the polymer micro particles may include a reaction product of a blocking compound containing isothiocyanate group and a polypeptide containing a reactive functional group.

That is, the polymer may include a polypeptide having a thiourea-based functional group in which an isothiocyanate group (-N=C=S) contained in the blocking compound containing isothiocyanate group, and a reactive functional group contained in a polypeptide containing a reactive functional group react to form a thiourea-based functional group.

Examples of the first crosslinking agent are not particularly limited. Specifically, the first crosslinking agent may include one of iron(III) chloride and aluminum chloride.

Meanwhile, the step of subjecting a mixture containing a combination of a polypeptide and a blocking compound, a biocompatible polymer, and a first crosslinking agent to a primary crosslinking reaction to form crosslinked particles may include a step of forming a mixed solution in which the polypeptide, the blocking compound, and the biocompatible polymer are dissolved; and a step of discharging the mixed solution and adding it to the first crosslinking agent.

That is, the first crosslinking reaction may be a liquid phase reaction proceeding by adding a first crosslinking agent to a mixed solution containing the combination of the polypeptide and the blocking compound, and a biocompatible polymer.

In addition, in the step of subjecting a mixture containing a combination of a polypeptide and a blocking compound, a biocompatible polymer, and a first crosslinking agent to a primary crosslinking reaction to form crosslinked particles, the primary crosslinking reaction may be an ionic crosslinking reaction.

That is, in the step of subjecting a mixture containing a combination of a polypeptide and a blocking compound, a biocompatible polymer, and a first crosslinking agent to a primary crosslinking reaction to form crosslinked particles, the primary crosslinking agent can be an ionic compound. In one embodiment of the invention, examples of the primary crosslinking agent are not particularly limited. Specifically, the primary crosslinking agent may include one of iron(III) chloride and aluminum chloride.

Meanwhile, in the step of extracting the crosslinked particles and performing a secondary crosslinking reaction in an organic solvent containing a second crosslinking agent, the crosslinked particles and the second crosslinking agent may be those that undergo a crosslinking reaction in a solid phase.

That is, according to the method for producing the polymer micro particles, a secondary crosslinking reaction that proceeds in a solid phase in an alkaline organic solvent can be included. Further, the secondary crosslinking reaction proceeding in the solid phase may mean a secondary crosslinking reaction that dehydrates the swollen polymer particles and progresses to a more aggregated state. Similarly to one embodiment of the present invention, when the secondary crosslinking reaction proceeds in a solid phase on an organic solvent is included, that is, when the secondary crosslinking reaction proceeds in a more aggregated state by dehydrating the swollen polymer particles, excellent crosslinking efficiency is realized as compared to the case that is not so, and thereby, micro particles having high strength can be obtained.

Recovering the crosslinked particles may be carried out by a method of using a mesh sieve having a sieve size of 30 $\mu$m or more. After recovering the crosslinked particles formed through the primary crosslinking reaction as described above, the particles are re-dispersed in an organic solvent and the second crosslinking agent is added, thereby allowing the secondary crosslinking reaction to proceed in a more aggregated solid phase.

Examples of the second crosslinking agent are not particularly limited. Specifically, the second crosslinking agent may include one selected from the group consisting of 1,4-butandiol diglycidyl ether (BDDE), ethylene glycol diglycidyl ether (EGDGE), 1,6-hexanediol diglycidyl ether, propylene glycol diglycidyl ether, polypropylene glycol diglycidyl ether, polytetramethylene glycol diglycidyl ether, neopentyl glycol diglycidyl ether, polyglycerol polyglycidyl ether, diglycerol polyglycidyl ether, glycerol polyglycidyl ether, tri-methylpropane polyglycidyl ether, 1,2-(bis(2,3-epoxypropoxy)ethylene, pentaerythritol polyglycidyl ether, sorbitol polyglycidyl ether, divinylsulfone and glutaraldehyde. More specifically, the second crosslinking agent may be 1,4-butandiol diglycidyl ether (BDDE).

The first crosslinking agent and the second crosslinking agent may each independently include at least one selected from the group consisting of iron(III) chloride, aluminum chloride, 1,4-butanediol diglycidyl ether, glutaraldehyde, ethylene glycol diglycidyl ether, hexanediol diglycidyl ether, propylene glycol diglycidyl ether, polyethylene glycol diglycidyl ether, polypropylene glycol diglycidyl ether, polytetramethylene glycol diglycidyl ether, neopentyl glycol diglycidyl ether, polyglycol polyglycidyl ether, diglycerol polyglycidyl ether, glycerol polyglycidyl ether, trimethylpropane polyglycidyl ether, bisepoxypropoxyethylene, pentaerythritol polyglycidyl ether, sorbitol polyglycidyl ether, and divinyl sulfone.

Further, the second crosslinking agent may be contained in an amount of 30 parts by weight or more and 300 parts by weight or less, 40 parts by weight or more and 250 parts by weight or less, or 50 parts by weight or more and 200 parts by weight or less with respect to 100 parts by weight of the biocompatible polymer. In the method for producing polymer micro particles according to an embodiment of the present invention, as the secondary crosslinking reaction proceeds after the primary crosslinking reaction as described above, the second crosslinking agent is added in an amount of 30 parts by weight or more and less than 300 parts by weight based on 100 parts by weight of the biocompatible polymer, thereby capable of producing polymer micro particles with sufficient mechanical strength and spheroidization ratio.

When the second crosslinking agent is added in an amount of less than 30 parts by weight based on 100 parts by weight of the biocompatible polymer, the degree of crosslinking is low, making it difficult to form particles, and when the second crosslinking agent is added in excess of 300 parts by weight, there may be a technical problem that an excessive amount of unreacted crosslinking agent is not removed and remains in the crosslinked particles.

Meanwhile, in the step of extracting the crosslinked particles and performing a secondary crosslinking reaction in an organic solvent containing a second crosslinking agent, the organic solvent may be at least one selected from the group consisting of ethanol, N,N-dimethylformamide, N,N-dimethylacetamide, N-methyl-2-pyrrolidone, N-methylcaprolactam, 2-pyrrolidone, N-ethylpyrrolidone, N-vinylpyrrolidone, dimethyl sulfoxide, tetramethylurea, pyridine, dimethyl sulfone, hexamethyl sulfoxide, gamma-butyrolactone, 3-methoxy-N,N-dimethylpropanamide, 3-ethoxy-N,N-dimethylpropanamide, 3-butoxy-N,N-dimethylpropanamide, 1,3-dimethyl-imidazolidinone, ethyl amyl ketone, methyl nonyl ketone, methyl ethyl ketone, methyl isoamyl ketone, methyl isopropyl ketone, cyclohexanone, ethylene carbonate, propylene carbonate, diglyme, 4-hydroxy-4-methyl-2-pentanone, ethylene glycol monomethyl ether, ethylene glycol monomethyl ether acetate, ethylene glycol monoethyl ether, ethylene glycol monoethyl ether acetate, ethylene glycol monopropyl ether, ethylene glycol monopropyl ether acetate, ethylene glycol monoisopropyl ether, ethylene glycol monoisopropyl ether acetate, ethylene glycol monobutyl ether, and ethylene glycol monobutyl ether acetate.

Meanwhile, the micro carrier of the one embodiment may further include a cell adhesion-inducing layer formed on the surface of the polymer micro particles. The cell adhesion-inducing layer is composed of cell adhesion materials, and allows adherent cells to be stably adhered, spread and cultured.

The cell adhesion-inducing layer may include one or more cell adhesion materials selected from the group consisting of gelatin, collagen, fibronectin, chitosan, polydopamine, poly L-lysine, vitronectin, peptide containing RGD, acrylic polymer containing RGD, lignin, cationic dextran and derivatives thereof. That is, the cell adhesion-inducing layer may include a cell adhesion material which includes one selected from the group consisting of gelatin, collagen, fibronectin, chitosan, polydopamine, poly L-lysine, vitronectin, peptide containing RGD, acrylic polymer containing RGD, lignin, cationic dextran and derivatives thereof, or a mixture of two or more thereof.

The cell adhesion-inducing layer may be formed on the surface of the polymer micro particles. That is, the cell adhesion-inducing layer may be in direct contact with the surface of the polymer micro particles, or the cell adhesion-inducing layer may be in contact with the surface of another layer that is in contact with the surface of the polymer micro particles.

In one example, the micro carrier includes a cell adhesion-inducing layer formed so as to directly contact the surface of the polymer micro particles, whereby by introducing a cell adhesion-inducing layer on the surface of the micro carrier, the degree of floating of the micro carrier in the culture medium can be adjusted, and the effect of stably adhering and culturing cells can be obtained.

The cell adhesion-inducing layer may have a layer thickness of 1 nm to 10,000 nm, or 10 nm to 1000 nm, or 50 nm to 500 nm, or 80 nm to 200 nm, wherein the layer thickness of the cell adhesion-inducing layer may be obtained by subtracting the radius of the polymer micro particles inside from the overall radius of the micro carrier. The radius means 1/2 value of the diameter. An example of a method for measuring the diameter is not particularly limited, but as an example, it can be measured through confocal fluorescence microscopy, electron transmission microscopy (TEM) or cross-sectional IR, cross-sectional SEM images.

The micro carrier may have an average diameter of 1 $\mu$m to 1,000 $\mu$m, or 10 $\mu$m to 1000 $\mu$m, or 100 $\mu$m to 1,000 $\mu$m, or 100 $\mu$m to 800 $\mu$m. Examples of the method for measuring the diameter are not particularly limited, but as an example, it may be measured through an optical microscope.

Meanwhile, the ratio of the radius of the polymer micro particle to the thickness of the cell adhesion-inducing layer may be 1:0.00001 to 1:0.1, or 1:0.0001 to 1:0.01.

When the ratio of the radius of the polymer micro particles to the thickness of the cell adhesion-inducing layer is exceeds 1:0.00001, the cell adhesion-inducing layer is too thin compared to the polymer micro particles and thus, it is likely to decrease the degree of adhesion between the cells and the micro carriers during cell culture. When the ratio less than 1:0.1, the cell adhesion-inducing layer becomes thicker as compared to the polymer micro particles, which may cause a problem of changing physical properties such as hydrophilicity of the polymer micro particles and thus lowering dispersibility.

The micro carrier may have a cell adhesion of 2000% or more, or 2500% or more, or 3000% or more, or 4000% or less, or 2000% to 4000%, or 2500% to 4000%, or 3000% to 4000% as calculated according to the following Equation.

Cell adhesion = (Number of cells after charging the micro carrier into the cell culture medium and culturing at 37°C for 7 days / Number of cells initially contained in the cell culture medium) X 100. [Equation]

The cell culture medium, cells, and culture conditions are not particularly limited, and conventionally known various cell culture media, cells, and culture conditions can be applied without limitation. Also, the method for measuring the number of cells is not particularly limited, and conventionally known various methods for measuring the number of cells can be applied without limitation.

As the micro carrier satisfies a cell adhesion of 2000% or more, 2500% or more, 3000% or more, 4000% or less, 2000% to 4000%, or 2500% to 4000%, or 3000% to 4000% as calculated according to the Equation, the cell adhesion of the micro carrier is improved, which increases the surface efficiency through three-dimensional expansion culture, and has the advantage of enabling large-scale expansion of adherent cells.

Meanwhile, when the micro carrier has a cell adhesion calculated according to the Equation that is less than the above range, there is a limit in that the cell adhesion on the surface of the micro carrier is weak, which makes it difficult to sufficiently proceed the three-dimensional expansion culture.

Meanwhile, the micro carrier may further include a primer polymer layer formed on the surface of the polymer micro particles.

That is, a mixed layer of one type of primer polymer layer and one type of cell adhesion-inducing layer may be further included on the surface of the polymer micro particles. In a mixed layer of one type of primer polymer layer and one type of cell adhesion-inducing layer, the laminating order of these is not particularly limited, and a structure in which a cell adhesion-inducing layer is laminated on a primer polymer layer or a structure in which a primer polymer layer is laminated on a cell adhesion-inducing layer is applicable.

Meanwhile, the primer polymer layer functions as an adhesive layer capable of introducing a functional polymer onto the surface of the polymer micro particles, whereby a polymer layer for cell adhesion on the surface of the micro carrier can be effectively introduced and stably maintained during culture.

Examples of the primer polymer layer are not particularly limited, but are catechol derivatives capable of inducing water phase adhesion, and may include any one or more selected from the group consisting of L-dihydroxyphenylalanine (L-DOPA), dopamine, polydopamine, norepinephrine, epinephrine, epigallocatechin and derivatives thereof.

The micro carrier may be a micro carrier for cell culture.

## 2. Cell Composite

According to another embodiment of the present invention, there can be provided a cell composite comprising: the micro carrier; and cells adhered onto the surface of the micro carrier. The contents concerning the micro carrier can include all the contents described above in the one embodiment.

In the cell composite of another embodiment, the composite can be formed in a state where the cells are stably attached to the micro carrier, thereby increasing the cell viability and realizing a high implantation rate within a body.

The cell is an adherent animal cell and examples thereof are not particularly limited, but for example, it may be fibroblasts, epithelial cell, osteoblast, chondrocyte, hepatocytes, human-derived cord blood cells, human bone marrow-derived mesenchymal stem cells, CHO (Chinese hamster ovary) cells, kidney cells (HEK293, BHK21, MDCK, vero cell, etc.), or a mixture of two or more thereof.

The density of the cells may be 1.02 g/$cm^3$ or more and less than 1.1 g/$cm^3$.

The cells may be attached onto the surface of the micro carrier. That is, the cell may directly contact the surface of the micro carrier, or the cell may contact the surface of another layer that is in contact with the surface of the micro carrier.

### 3. Medical Composition

According to yet another embodiment of the present invention, there can be provided a medical composition comprising the micro carrier of the one embodiment or the cell composite of the other embodiment. The contents concerning the micro carrier can include all the contents described above in the one embodiment. The contents concerning the cell composite can include all the contents described above in the one embodiment.

That is, when the pharmaceutically active material of the one embodiment is applied for medical purposes, a medical composition can be provided.

In such as case, examples of the medically effective material are not particularly limited, and depending on the application use of the polymer micro particles of the one embodiment, an effective material suitable for the use can be applied without limitation. That is, specific examples of the medically effective material are not limited, and may include polyglycine, polyalanine, polyvaline, polyleucine, polyisoleucine, polyphenylalanine, polytryptophan, polyglutamic acid, polyproline, polyaspartic acid, and the like, or may include a medically effective material such as an elastin-derived polypeptide (repetitive polymer of (valine-proline-glycine-valine-glycine)), and all materials with proven medical efficacy can be applied without limitation.

The addition amount of the medically effective material is also not particularly limited, and the content can be used without limitation depending on the application purpose and object. For example, the medically effective material may be contained in an amount of 0.0001 parts by weight or more and 1000 parts by weight or less based on 100 parts by weight of the polymer micro particles, which can be contained without limitation in a small amount or an excess amount relative to the polymer micro particles.

### 4. Cosmetic Composition

According to another embodiment of the present invention, there can be provided a cosmetic composition comprising the micro carrier of the one embodiment or the cell composite of the other embodiment. The contents concerning the micro carrier can include all the contents described above in the one embodiment. The contents concerning the cell composite can include all the contents described above in the one embodiment.

That is, when the pharmaceutically active material of the one embodiment is applied for cosmetic purposes, a cosmetic composition can be provided.

In such as case, examples of the medically effective material are not particularly limited, and depending on the application use of the polymer micro particles of the one embodiment, an effective material suitable for the use can be applied without limitation. That is, specific examples of the cosmetically effective material are not limited, and may include natural extracts, proteins, vitamins, enzymes, antioxidants, and the like, and all materials with proven cosmetic efficacy can be applied without limitation.

The addition amount of the cosmetically effective material is also not particularly limited, and the content can be used without limitation depending on the application purpose and object. For example, the cosmetically effective material may be contained in an amount of 0.0001 parts by weight or more and 1000 parts by weight or less based on 100 parts by weight of the polymer micro particles, which can be contained without limitation in a small amount or an excess amount relative to the polymer micro particles.

### 5. Medical Article

According to yet another embodiment of the present invention, there can be provided a medical article comprising the medical composition of the other embodiment. The contents concerning the medical composition can include all the contents described above in the one embodiment.

Examples of the medical article are not particularly limited, but in order to realize the characteristics of the present invention, it is suitable when the medical article is used by inserting it into the body or when strength must be maintained for a long period of time, and for example, a body implant, a body insertion type drug delivery system, a transdermal patch, a wound healing agent, and the like can be mentioned.

**6. Cosmetic Article**

According to yet another embodiment of the present invention, there can be provided a cosmetic article comprising the cosmetic composition of the other embodiment of the present invention. The contents concerning the cosmetic composition can include all the contents described above in the one embodiment.

Examples of the cosmetic article are not particularly limited, but in order to realize the characteristics of the present invention, and for example, beauty creams, lotions, hair gels, packs, and the like may be mentioned.

The structure of the cosmetic pack is not particularly limited, but for example, it may include a support, and a cosmetically effective material delivery layer formed on the support and including the polymer micro particles of the other embodiment. Examples of the support include woven fabric, nonwoven fabric, silicone, polyethylene terephthalate, polyethylene, polypropylene, polyurethane, metal mesh, polyester, and the like.

**[Advantageous Effects]**

According to the present invention, there can be provided a micro carrier that not only can achieve stability together with high crosslinking efficiency and production yield, but also can confirm drug release properties without chemical modification of protein or peptide pharmaceutical effective materials, can be injected into the body immediately after 3D culture without a cell detachment process and can provide a stable environment for adherent cells, thereby increasing cell viability and contributing to a high implantation rate in the body, and a cell composite, medical composition, cosmetic composition, medical articles and cosmetic articles using the same.

**[DETAILED DESCRIPTION OF THE EMBODIMENTS]**

Hereinafter, the present invention will be described in more detail by way of examples. However, these examples are provided for illustrative purposes only and are not intended to limit the scope of the present invention.

**<Example: Production of polymer micro particles and micro carriers for cell culture>**

Example 1

(1) Production of polymer micro particles

0.15 g of hyaluronate(salt of hyaluronic acid, weight average molecular weight: 500 kDa) and 1 g of gelatin (gel strength: 300 g) were dissolved in distilled water at a concentration of 1.0 wt.% and 12.5 wt.%, respectively, to prepare solutions, and these two solutions were mixed in a 1:1 volume ratio. An aqueous solution of fluorescein isothiocyanate-polyproline (hydrodynamic radius: 2.5 nm, reactive functional group content: 0.01 mol/$10^3$ g) was mixed therewith at a concentration of 600 $\mu$g/mL. The mixed solution (4 g) was discharged through the nozzle of the Buchi encapsulator and solidified in 5wt% $FeCl_3$ (iron chloride) solution to produce particles. Then, the solid particles were washed 5 times in ethanol, and then 0.5 ml (0.55 g) of 1,4-butandiol diglycidyl ether (BDDE) as a crosslinking agent was added to 25 g of the solid particle dispersion, and subjected to a crosslinking reaction at room temperature for 4 days. Then, the crosslinked particles produced by washing with ethanol and using a mesh sieve having a sieve size of 45 $\mu$m were recovered.

(2) Production of micro carriers for cell culture

The recovered particles were immersed in tris buffer (pH 8.0) in which dopamine was dissolved at 1 mg/mL, and coated under stirring at room temperature for 2 hours. After washing excess coating material with ethanol, the particles were filtered on a 45 $\mu$m sieve, and then used as a micro carrier for cell culture.

Example 2

Polymer micro particles and micro carriers for cell culture were produced in the same manner as in Example 1, except that in (1) of Example 1, 1,4-butanediol diglycidyl ether was added in the content of 1 ml (1.1 g).

Example 3

Polymer micro particles and micro carriers for cell culture were produced in the same manner as in Example 1, except that in (1) of Example 1, the size of the nozzle was changed to 200 $\mu$m.

Example 4

Polymer micro particles and micro carriers for cell culture were produced in the same manner as in Example 1, except that in (1) of Example 1, the size of the nozzle was changed to 200 $\mu$m, and 1,4-butanediol diglycidyl ether was added in the content of 1 ml (1.1 g).

Example 5

Polymer micro particles and micro carriers for cell culture were produced in the same manner as in Example 1, except that in (1) of Example 1, the size of the nozzle was changed to 300 $\mu$m.

Example 6

Polymer micro particles and micro carriers for cell culture were produced in the same manner as in Example 1, except that in (1) of Example 1, the size of the nozzle was changed to 300 $\mu$m, and 1,4-butanediol diglycidyl ether was added in the content of 1 ml (1.1 g).

Example 7

The crosslinked particles produced in (1) of Example 1 were used as micro carriers for cell culture, without proceeding the dopamine coating of (2) of Example 1.

Example 8

Micro carriers for cell culture were produced in the same manner as in Example 7, except that 1,4-butanediol diglycidyl ether was added in the content of 1 ml (1.1 g).

Example 9

Micro carriers for cell culture were produced in the same manner as in Example 7, except that the size of the nozzle was changed to 200 $\mu$m.

Example 10

Micro carriers for cell culture were produced in the same manner as in Example 7, except that the size of the nozzle was changed to 200 $\mu$m, and 1,4-butanediol diglycidyl ether was added in the content of 1 ml (1.1 g).

Example 11

Micro carriers for cell culture were produced in the same manner as in Example 7, except that the size of the nozzle was changed to 300 $\mu$m.

Example 12

Micro carriers for cell culture were produced in the same manner as in Example 7, except that the size of the nozzle was changed to 300 $\mu$m, and 1,4-butanediol diglycidyl ether was added in the content of 1 ml (1.1 g).

<Comparative Example>

Comparative Example 1: Production of polymer micro particles

0.15 g of hyaluronate (weight average molecular weight: 500 kDa) and 1 g of gelatin (gel strength: 300 g) were dissolved in distilled water at a concentration of 1.5 wt.% and 10 wt.%, respectively, to prepare solutions, and these two solutions were mixed in a 1:1 volume ratio. An aqueous solution of fluorescein isothiocyanate-polyproline (hydrodynamic radius: 1.7 nm, reactive functional group content: 1.1 mol/$10^3$ g) was mixed thereto at a concentration of 600 $\mu$g/mL. The mixed solution (4 g) was mixed with a liquid paraffin solution (40 g) to prepare a mixed solution containing a water-in-oil (W/O) micro emulsion. Then, 1.1 g (1 ml) of 1,4-butandiol diglycidyl ether (BDDE) as a crosslinking agent was added to 44 g of the mixed solution, subjected to a crosslinking reaction at room temperature for 4 days, and then washed with acetone, dichloromethane, and ethanol in that order to produce crosslinked particles. At this time, the crosslinked particles produced using a mesh sieve having a sieve size of 45 $\mu$m were recovered.

Comparative Example 2: Production of polymer micro particles

Polymer micro particles were produced in the same manner as in Comparative Example 1, except that in Comparative Example 1, human growth hormone (hydrodynamic radius: 2.5 nm, reactive functional group content: 10 mol/$10^3$ g) was used instead of fluorescein isothiocyanate-polyproline as an active material.

Comparative Example 3: Production of polymer micro particles

Polymer micro particles were produced in the same manner as in Comparative Example 1, except that in Comparative Example 1, bovine serum albumin (hydrodynamic radius: 3.8 nm, reactive functional group content: 20 mol/$10^3$ g) was used instead of fluorescein isothiocyanate-polyproline as an active material.

Comparative Example 4: Production of polymer micro particles

Polymer micro particles were produced in the same manner as in Comparative Example 1, except that in Comparative Example 1, fluorescein isothiocyanate-polylysine (hydrodynamic radius: 1.9 nm, reactive functional group content: 50 mol/$10^3$ g) was used instead of fluorescein isothiocyanate-polyproline as an active material.

Comparative Example 5: Production of polymer micro particles

Polymer micro particles were produced in the same manner as in (1) of Example 1, except that in (1) of Example 1, fluorescein isothiocyanate-polylysine (hydrodynamic radius: 1.9 nm, reactive functional group content: 50 mol/$10^3$ g) was used instead of fluorescein isothiocyanate-polyproline as an active material.

Comparative Example 6: Production of micro carriers for cell culture

The polymer micro particles obtained in (1) of Example 1 were used as micro carriers for cell culture.

[Table 1]

| Category | BDDE:HA molar ratio | Type of pharmaceutically effective material | Amount of pharmaceutically effective material |
|---|---|---|---|
| Example 1, 7 | 15:1 | FITC-(poly-proline) | 1200 ng |
| Example 2, 8 | 30:1 | FITC-(poly-proline) | 1200 ng |
| Example 3, 9 | 15:1 | FITC-(poly-proline) | 1200 ng |
| Example 4, 10 | 30:1 | FITC-(poly-proline) | 1200 ng |
| Example 5, 11 | 15:1 | FITC-(poly-proline) | 1200 ng |
| Example 6, 12 | 30:1 | FITC-(poly-proline) | 1200 ng |
| Comparative Example 1 | 30:1 | FITC-(poly-proline) | 100 ng |
| Comparative Example 2 | 30:1 | Human growth hormone | 100 ng |
| Comparative Example 3 | 30:1 | Bovine serum albumin | 100 ng |
| Comparative Example 4 | 30:1 | FITC-(poly-L-lysine) | 100 ng |

(continued)

| Category | BDDE:HA molar ratio | Type of pharmaceutically effective material | Amount of pharmaceutically effective material |
|---|---|---|---|
| Comparative Example 5 | 30:1 | FITC-(poly-L-lysine) | 100 ng |
| * BDDE: 1,4-Butandiol diglycidyl ether<br>* HA: hyaluronate (weight average molecular weight: 500 kDa, available from SK Bioland)<br>* FITC: Fluorescein isothiocyanate | | | |

**<Experimental Example 1>**

For the polymer micro particles produced in Examples and Comparative Examples, the physical properties of the polymer micro particles were measured by the following manner, and are described in Tables 2 and 3 below.

**1. Average Diameter and Swelling Degree**

The average diameter of the polymer micro particles of Examples and Comparative Examples was measured, and the swell degree was calculated therefrom.

The swelling degree of the particles according to the present invention was calculated according to the following Equation 1.

Swelling degree = { (Average diameter in distilled water)$^3$ - (Average diameter of dried particles)$^3$} / (Average diameter of dried particles)$^3$. [Equation 1]

At this time, it means that as the swelling degree value is closer to 0, swelling does not occur, and it means that as the value becomes large, the particles swell and become large.

The average diameter of the dried particles and the average diameter in distilled water were measured using an optical microscope (Olympus, BX53).

**2. Spheroidization Degree**

Optical (Olympus, BX53) photographs of the polymer micro particles of Examples and Comparative Examples were taken, and the spheroidization degree was calculated therefrom.

The spheroidization degree according to the present invention was calculated as the average value of the ratio of the longest diameter to the shortest diameter (length-diameter ratio) of 30 arbitrary particles in an optical photograph.

At this time, it means that as the spheroidization degree value is closer to 100, it is closer to a sphere.

[Table 2]

| Category | Average diameter of dried particles ($\mu$m) | Average diameter in distilled water ($\mu$m) | Swelling degree | Spheroidization degree |
|---|---|---|---|---|
| Example 1, 7 | 200 | 310 | 2.7 | 80 |
| Example 2, 8 | 234 | 330 | 1.8 | 80 |
| Example 3, 9 | 194 | 322 | 3.5 | 80 |
| Example 4, 10 | 248 | 434 | 4.3 | 80 |
| Example 5, 11 | 354 | 446 | 0.9 | 80 |
| Example 6, 12 | 588 | 804 | 1.5 | 80 |
| Comparative Example 1 | 500 | 1250 | 2.5 | 90 |
| Comparative Example 2 | 510 | 1240 | 2.4 | 90 |
| Comparative Example 3 | 504 | 1190 | 2.3 | 90 |
| Comparative Example 4 | 505 | 1200 | 2.37 | 90 |
| Comparative Example 5 | 510 | 1210 | 2.37 | 80 |

As shown in Table 2, it could be confirmed that in the polymer micro particles of Examples, which were produced by the production method the present invention comprising the step of recovering the particles after the primary crosslinking and subjecting them to a secondary crosslinking, the degree of spheroidization appears to be 80 or more, indicating a high degree of spheroidization, and at the same time, the degree of swelling appears to be 0.9 or more and 4.3 or less, indicating an excellent degree of crosslinking.

**3. Release Amount**

At each release condition shown in Table 3 below, the polymer micro particles of Examples and Comparative Examples were dispersed at 5 mg/mL, and then cultured at 37°C for 4 weeks. Fluorescence intensity of the culture supernatant was measured at a wavelength of 495 nm using a Synergy HTX multi-mode plate reader device (BioTek), and the fluorescein isothiocyanate-polypeptide content was calculated through the calibration curve of the combination of the polypeptide and the blocking compound (fluorescein isothiocyanate) used in the Examples and Comparative Examples.

[Table 3]

| Category | Release condition | Release period | Release amount (%) |
|---|---|---|---|
| Example 1, 7 | pH 7.4, 37 °C | 150 hr | 100 |
| Example 2, 8 | pH 7.4, 37 °C | 150 hr | 100 |
| Example 3, 9 | pH 7.4, 37 °C | 150 hr | 100 |
| Example 4, 10 | pH 7.4, 37 °C | 150 hr | 100 |
| Example 5, 11 | pH 7.4, 37 °C | 150 hr | 100 |
| Example 6, 12 | pH 7.4, 37 °C | 150 hr | 100 |
| Comparative Example 1 | pH 7.4, 37 °C | 4-week | 17 |
| Comparative Example 2 | pH 7.4, 37 °C | 4-week | 0 |
| Comparative Example 3 | pH 7.4, 37 °C | 4-week | 0 |
| Comparative Example 4 | pH 7.4, 37 °C | 4-week | 0 |
| Comparative Example 5 | pH 7.4, 37 °C | 150 hr | 0 |

As shown in Table 3, it could be confirmed that the polymer micro particles of Examples, which were produced by the production method of the present invention comprising the step of recovering the particles after a primary crosslinking and subjecting them to a secondary crosslinking, exhibited the release amount of 100% during the release period of 150 hours or more, showing the protein drug release properties without chemical modification of the pharmaceutically active material. Meanwhile, it could be confirmed that the polymer micro particles of Comparative Examples that have undergone only the primary crosslinking exhibited a release amount of 20% or less during a release period of 150 hours or more, showing very poor protein drug release properties due to chemical modification of the pharmaceutically active material.

**<Experimental Example 2>**

The physical properties of the micro carriers for cell culture produced in Example 1 and Comparative Example 6 were measured by the following method and are described in Table 4 below.

**4. Thickness of Coating layer**

The thickness of the coating layer formed on the surface of the polymer micro particles in the micro carrier for cell culture was measured through a cross-sectional TEM image.

**5. Cell Adhesion**

A culture medium containing mesenchymal stem cells (density: 1.05 g/$cm^3$) was filled in a 100 mL vertical wheel bioreactor (PBS), and the micro carrier for cell culture was injected into the culture medium and stirred. After culturing at 37°C for 7 days, the number of cells cultured in a cell culture micro carrier was confirmed. Then, the cell adhesion of the micro carrier was evaluated by comparing it with the number of initially injected cells as shown in the following Equation.

Cell adhesion = (Number of cells after charging the micro carrier into the cell culture medium and culturing at 37°C for 7 days / Number of cells initially contained in the cell culture medium) X 100.

[Table 4]

| Category | Coating layer thickness ($\mu$m) | Cell adhesion (%) |
|---|---|---|
| Example 1 | 0.1 | 3000 |
| Comparative Example 6 | No coating layer | 1700 |

As shown in Table 4, it could be confirmed that as the micro carrier for cell culture of Example 1 includes a cell adhesion material coating layer of 0.1 $\mu$m on the surface of the polymer micro particles, the cell adhesion is remarkably improved as compared to Comparative Example 6.

## Claims

**1.** A micro carrier comprising polymer micro particles which comprises: a polymer matrix containing a biocompatible polymer; and a polypeptide dispersed in the polymer matrix and having a reactive functional group content of 1 mol/$10^3$ g or less,

wherein the biocompatible polymer comprises hyaluronic acid and gelatin,
wherein the polypeptide having the reactive functional group content of 1 mol/$10^3$ g or less comprises a polypeptide in which 90 mol% or more of reactive functional groups on the surface are substituted with a blocking compound,
wherein the reactive functional group is an amine group,
wherein the reactive functional group content is calculated according to the following Equation,

Reactive amine functional group content (mol/$10^3$ g) = Amount of hydrochloric acid added (L) x Molarity of aqueous hydrochloric acid solution (mol/L) / Amount of polypeptide dissolved in solvent ($10^3$ g) [Equation]

**2.** The micro carrier according to claim 1 wherein:
the polypeptide has a hydrodynamic radius of 1 nm to 30 nm.

**3.** The micro carrier according to claim 1 wherein:
the polypeptide comprises a polypeptide having a functional group that is a combination of a blocking compound and a reactive functional group.

**4.** The micro carrier according to claim 1 wherein:
the polymer micro particles have a swelling degree of 10 or less according to the following Equation 1:

Swelling degree = {(Average diameter in distilled water)$^3$ - (Average diameter of dried particles)$^3$} / (Average diameter of dried particles)$^3$. [Equation 1]

**5.** The micro carrier according to claim 1 wherein:
the polymer micro particles has a release amount of the pharmaceutically active material under release conditions of pH 5.0 or more and 30°C or more and 40° C or less after culturing for more than 150 hours or more, of 20% or more and 100% or less.

**6.** The micro carrier according to claim 1 wherein:

the polymer matrix comprises a first crosslinking region in which the biocompatible polymer is crosslinked via a first crosslinking agent; and

a second crosslinking region in which the biocompatible polymer is crosslinked via a second crosslinking agent.

**7.** The micro carrier according to claim 1, further comprising,

a cell adhesion-inducing layer formed on the surface of the polymer micro particles, and
the cell adhesion-inducing layer comprises one or more cell adhesion materials selected from the group consisting of gelatin, collagen, fibronectin, chitosan, polydopamine, poly L-lysine, vitronectin, peptide containing RGD, acrylic polymer containing RGD, lignin, cationic dextran and derivatives thereof.

**8.** The micro carrier according to claim 1, wherein:
the micro carrier has a cell adhesion of 2000% or more as calculated according to the following Equation:

Cell adhesion = (Number of cells after charging the micro carrier into the cell culture medium and culturing at 37°C for 7 days / Number of cells initially contained in the cell culture medium) X 100%. [Equation]

**9.** A cell composite comprising:

the micro carrier of claim 1; and
cells adhered onto the surface of the micro carrier.

**10.** A medical composition comprising any one of the micro carrier of claim 1 or the cell composite of claim 9.

**11.** A cosmetic composition comprising any one of the micro carrier of claim 1 or the cell composite of claim 9.

**12.** A medical article comprising the medical composition of claim 10, or a cosmetic article comprising the cosmetic composition of claim 11.

## Patentansprüche

**1.** Mikroträger, der Polymermikropartikel umfasst, umfassend: eine Polymermatrix, die ein biokompatibles Polymer enthält; und ein Polypeptid, das in der Polymermatrix dispergiert ist und einen Gehalt an reaktiven funktionellen Gruppen von 1 mol/$10^3$ g oder weniger aufweist,

wobei das biokompatible Polymer Hyaluronsäure und Gelatine umfasst,
wobei das Polypeptid mit einem Gehalt an reaktiven funktionellen Gruppen von 1 mol/$10^3$ g oder weniger ein Polypeptid umfasst, bei dem 90 mol% oder mehr der reaktiven funktionellen Gruppen an der Oberfläche durch eine blockierende Verbindung substituiert sind,
wobei die reaktive funktionelle Gruppe eine Amingruppe ist,
wobei der Gehalt an reaktiven funktionellen Gruppen nach der folgenden Gleichung berechnet wird:

Gehalt an reaktiven funktionellen Amingruppen (mol/$10^3$ g) = Menge der zugesetzten Salzsäure (L) $\times$ Molarität der wässrigen Salzsäurelösung (mol/l) / Menge des in Lösungsmittel gelösten Polypeptids ($10^3$ g). [Gleichung]

**2.** Mikroträger nach Anspruch 1, wobei:
das Polypeptid einen hydrodynamischen Radius von 1 nm bis 30 nm aufweist.

**3.** Mikroträger nach Anspruch 1, wobei:
das Polypeptid ein Polypeptid mit einer funktionellen Gruppe umfasst, die eine Kombination aus einer blockierenden Verbindung und einer reaktiven funktionellen Gruppe ist.

**4.** Mikroträger nach Anspruch 1, wobei:
die Polymermikropartikel einen Quellgrad von 10 oder weniger nach der folgenden Gleichung 1 aufweisen Quellgrad = {(durchschnittlicher Durchmesser in destilliertem Wasser)$^3$ - durchschnittlicher Durchmesser der getrockneten Partikel)$^3$ / (durchschnittlicher Durchmesser der getrockneten Partikel)$^3$ [Gleichung 1]

5. Mikroträger nach Anspruch 1, wobei:
die Polymermikropartikel eine Freisetzungsmenge des pharmazeutisch wirksamen Materials unter Freisetzungsbedingungen von pH 5,0 oder mehr und 30°C oder mehr und 40°C oder weniger nach einer Kultivierung für mehr als 150 Stunden oder mehr von 20 % oder mehr und 100 % oder weniger aufweisen.

6. Mikroträger nach Anspruch 1, wobei:

die Polymermatrix einen ersten Vernetzungsbereich umfasst, in dem das biokompatible Polymer über ein erstes Vernetzungsmittel vernetzt ist; und
einen zweiten Vernetzungsbereich, in dem das biokompatible Polymer über ein zweites Vernetzungsmittel vernetzt ist.

7. Mikroträger nach Anspruch 1, ferner umfassend:

eine Zelladhäsions-induzierende Schicht, die auf der Oberfläche der Polymermikropartikel gebildet ist, und die Zelladhäsions-induzierende Schicht umfasst ein oder mehrere Zelladhäsionsmaterialien, ausgewählt aus der Gruppe bestehend aus Gelatine, Kollagen, Fibronektin, Chitosan, Polydopamin, Poly-L-Lysin, Vitronectin, RGD-haltigem Peptid, RGD-haltigem Acrylpolymer, Lignin, kationischem Dextran und Derivaten davon.

8. Mikroträger nach Anspruch 1, wobei:
der Mikroträger eine Zelladhäsion von 2000 % oder mehr aufweist, berechnet nach der folgenden Gleichung:

Zelladhäsion = (Anzahl der Zellen nach Einbringen des Mikroträgers in das Zellkulturmedium und 7-tägiger Kultivierung bei 37 °C / Anzahl der ursprünglich im Zellkulturmedium enthaltenen Zellen) $\times$ 100 %. [Gleichung]

9. Zellverbundstoff, umfassend:

den Mikroträger nach Anspruch 1; und
an der Oberfläche des Mikroträgers anhaftende Zellen.

10. Medizinische Zusammensetzung, umfassend den Mikroträger nach Anspruch 1 oder den Zellverbundstoff nach Anspruch 9.

11. Kosmetische Zusammensetzung, umfassend den Mikroträger nach Anspruch 1 oder den Zellverbundstoff nach Anspruch 9.

12. Medizinischer Artikel, umfassend die medizinische Zusammensetzung nach Anspruch 10, oder kosmetischer Artikel, umfassend die kosmetische Zusammensetzung nach Anspruch 11.

## Revendications

1. Micro-support comprenant des microparticules de polymère qui comprend : une matrice polymère contenant un polymère biocompatible ; et un polypeptide dispersé dans la matrice polymère et ayant une teneur en groupes fonctionnels réactifs de 1 mole/$10^3$ g ou moins, dans lequel le polymère biocompatible comprend de l'acide hyaluronique et de la gélatine,

dans lequel le polypeptide ayant une teneur en groupes fonctionnels réactifs de 1 mole/$10^3$ g ou moins comprend un polypeptide dans lequel 90 % en mole ou plus des groupes fonctionnels réactifs à la surface sont substitués avec un composé de blocage,

dans lequel le groupe fonctionnel réactif est un groupe amine,
dans lequel la teneur en groupes fonctionnels réactifs est calculée selon l'équation suivante, Teneur en groupes fonctionnels réactifs type amine (mole/$10^3$ g) = Quantité d'acide chlorhydrique ajoutée (L) x Molarité de la solution aqueuse d'acide chlorhydrique (mol/L)/Quantité de polypeptide dissous dans le solvant ($10^3$ g) [Equation]

2. Micro-support selon la revendication 1, dans lequel :
le polypeptide présente un rayon hydrodynamique de 1 nm à 30 nm.

3. Micro-support selon la revendication 1, dans lequel :
le polypeptide comprend un polypeptide ayant un groupe fonctionnel qui est une combinaison d'un composé de blocage et d'un groupe fonctionnel réactif.

4. Micro-support selon la revendication 1, dans lequel :
les microparticules de polymère présentent un degré de gonflement de 10 ou moins selon l'Équation 1 suivante :

Degré de gonflement = {(Diamètre moyen dans l'eau distillée)$^3$ - (Diamètre moyen des particules sèches)$^3$ }/(Diamètre moyen des particules sèches)$^3$. [Equation 1]

5. Micro-support selon la revendication 1, dans lequel :
les microparticules de polymère présentent une quantité de libération de matière pharmaceutiquement active, dans des conditions de libération de pH 5,0 ou plus et à 30 °C ou plus et 40 °C ou moins, après une culture de plus de 150 heures ou plus, de 20 % ou plus et 100 % ou moins.

6. Micro-support selon la revendication 1, dans lequel :

la matrice polymère comprend une première région de réticulation dans laquelle le polymère biocompatible est réticulé via un premier agent de réticulation ; et
une seconde région de réticulation dans laquelle le polymère biocompatible est réticulé via un second agent de réticulation.

7. Micro-support selon la revendication 1, comprenant en outre :

une couche d'induction d'adhérence cellulaire formée à la surface des microparticules de polymère, et
la couche d'induction d'adhérence cellulaire comprend une ou plusieurs matières d'adhérence cellulaire choisies dans le groupe constitué de la gélatine, du collagène, de la fibronectine, du chitosane, d'une polydopamine, d'une poly-L-lysine, de la vitronectine, d'un peptide contenant RGD, d'un polymère acrylique contenant RGD, de la lignine, d'un dextrane cationique et les dérivés de ceux-ci.

8. Micro-support selon la revendication 1, dans lequel :
le micro-support présente une adhérence cellulaire de 2000 % ou plus telle que calculée selon l'équation suivante :

Adhésion cellulaire = (Nombre de cellules après chargement du micro-support dans le milieu de culture cellulaire et culture à 37 °C pendant 7 jours/Nombre de cellules initialement contenues dans le milieu de culture cellulaire) X 100 %. [Equation]

9. Composite cellulaire comprenant :

le micro-support selon la revendication 1 ; et
des cellules adhérant à la surface du micro-support.

10. Composition médicale comprenant l'un quelconque parmi le micro-support selon la revendication 1 ou le composite cellulaire selon la revendication 9.

**11.** Composition cosmétique comprenant l'un quelconque parmi le micro-support selon la revendication 1 ou le composite cellulaire selon la revendication 9.

**12.** Article médical comprenant la composition médicale selon la revendication 10, ou article cosmétique comprenant la composition cosmétique selon la revendication 11.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020210129845
- KR 20210037578